(19) Europäisches Patentamt
European Patent Office
Office européen des brevets

(11) **EP 1 407 816 B1**

(12) **EUROPEAN PATENT SPECIFICATION**

(45) Date of publication and mention
of the grant of the patent:
**21.05.2008 Bulletin 2008/21**

(51) Int Cl.:
*B01J 19/00* (2006.01)     *C12Q 1/68* (2006.01)

(21) Application number: **02020812.0**

(22) Date of filing: **17.09.2002**

(54) **Method for arranging a polymer molecule**

Verfahren zum Anordnen einem Polymer-Molekül

Méthode pour mettre en place une molécule de polymère

(84) Designated Contracting States:
**AT BE BG CH CY CZ DE DK EE ES FI FR GB GR IE IT LI LU MC NL PT SE SK TR**

(43) Date of publication of application:
**14.04.2004 Bulletin 2004/16**

(73) Proprietor: **Kalachev, Alexey**
**12489 Berlin (DE)**

(72) Inventors:
- **Rabe, Prof. Dr. Jürgen**
  **14163 Berlin (DE)**
- **Severin, Dr. Nikolai**
  **12439 Berlin (DE)**
- **Kalachev, Dr. Alexey**
  **12489 Berlin (DE)**

(74) Representative: **Liesegang, Eva et al**
**Forrester & Boehmert,**
**Pettenkoferstrasse 20-22**
**80336 München (DE)**

(56) References cited:
WO-A-97/06278     US-A- 5 079 169
US-A- 5 314 829     US-A- 5 612 181
US-A- 5 677 126     US-A- 5 851 769

- HIDENOBU NAKAO ET AL.: "Development of Novel Polymer-coated Substrates for Straightening and Fixing DNA" NANO LETTERS, vol. 2, no. 5, 1992, pages 475-479, XP002225163
- HU, J. ET AL: "Artificial DNA Patterns by Mechanical Nanomanipulation" NANO LETTERS, vol. 2, no. 1, January 2002 (2002-01), pages 55-57,
- OUYANG, Z-Q ET AL.: "Molecular patterns by manipulating DNA molecules" J. VAC. SCI. TECHNOL., vol. 15, no. 4, 1997, pages 1385-1387,
- PODTELEZHNIKOV, A. A. ET AL.: "Dynamics of small loops in DNA molecules" MACROMOLECULES, vol. 33,
- Retrieved from the Internet: URL:http://en.wikipedia.org/wiki/Ideal_cha in> [retrieved on 2007-04-22]

**Description**

The present invention relates to a method for arranging a polymer molecule such as a synthetic polymer and macromolecule with biological activity (biomolecule), especially deoxyribonucleic acid (DNA), RNA, polysaccharides or proteins on a support.

Controlling and manipulating conformation and position of polymer molecules with nanometric resolution on surface represents a major industrial challenge in the field of nanotechnology, for example in sensors or controlled molecular assemblies and molecular electronic devices, or alternatively in problems of detection and analysis, for example gene probe analysis (cf. U.S. 6,376,177).

It may be useful, especially in case of molecular devices, to have not only straight (linear) molecular conformations but the opportunity to arrange any desired conformation of a polymer molecule and to achieve an exact positioning of polymer molecules with respect to each other on the surface. Synthetic organic compounds such as aromatic dendrimers were manipulated on the surface for this purpose (L. Shu et al, Angew. Chem. 113 (2001) 4802).

With respect to polymers which have coiled or helical conformation stabilized by intramolecular bonds such as hydrogen bonds, for example ds-DNA, it would be useful to have the ability to over-stretch the molecular chain on the surface to facilitate direct analysis of single polymer chain sequence (R.H. Austin et al., Stretch genes, Physics Today, 2 (1997) 32-38).

One of the most interesting molecular objects to be arranged on the surface at nanometric scale is DNA. Studies of DNA on the genetic level are progressing dramatically along with genetic engineering and molecular biology. DNA is the fundamental material in life science. In polymer science it is regarded as a naturally occurring and highly specific functional biopolymer with a diameter of the main chain around 2 nm which has a polymer unit (base) information every 0,3 nm. Different efforts have been made for manipulating and fixing arrangements of DNA for experimental studies. Most investigations in this area are concerned with the study of DNA molecules situated in a volume of solution or hydrogels i.e. when main part of investigated DNA is supported in dissolved state. In this way DNA -electrophoresis was performed on a specially structured chip (W.D. Volkmuth, R.H. Austin, Nature 358 (1992) 600) and only partial orientation of molecules in parallel to the electrical field was observed.

In order to stretch DNA for experimental investigations micro-beads have been chemically attached to one end of DNA placed in a fluid chamber (see for example Smith et al., Science 258 (1992) 1122). The other end of DNA can be also fixed. Afterwards a mechanical, magnetic or other field is applied to the bead to stretch DNA. However, as mentioned above, these methods are concerned with DNA manipulation in liquid volume, and they do not allow DNA manipulation and arrangement on a surface. From U.S. 5,851,769 methods are known for the physical mapping and positional cloning of genomic DNA. According to the known methods macromolecules are arranged prior to adsorption on surface. Manipulation of a polymer molecule is performed in solution by displacement of a nano-particle chemically attached to one end of the macromolecule and suspended together with the DNA-filament.

An attempt to arrange DNA on a surface was performed by deposition of molecules on the surface through the water removal with followed fabrication of a variety of DNA network structures by organic solvent treatment such as ethanol treatment (T. Kanno et al., Appl. Phys. Lett., 77 (2000) 3848). This method, however, is rather usable for preparing a DNA-containing aggregated film and does not allow for manipulation of single polymer molecules.

U.S. 6,303,296 discloses a method for aligning DNA on a surface of a support such as modified glass, wherein one end of DNA is anchored to the surface and the rest of the molecule is dissolved in an aqueous medium. Subsequently the liquid is removed through the displacing by gas (air) and the anchored DNA is subject to a gas-liquid-surface meniscus movement. In result DNA molecules are elongated and oriented perpendicular to the meniscus line. This method is referred to as "Molecular combing". This method was also applied for atomically flat substrates such as mica. Several attempts have been made to optimize molecular combing, for example by use of moving droplets and coating of a support surface on which DNA is anchored (Nakao et. al., Nano Letters 2 (2002) 475). Molecular combing" can provide only a linear conformation of the aligned polymer. It does not allow positioning single molecules with respect to each other. The over-stretching of DNA was not observed upon application of the molecular combing method, because mechanical forces developed by moved meniscus are relatively weak. Molecular combing does not allow the manipulations of a single polymer (DNA ) molecule. Being once bound from the solution to the surface of support and dried, the DNA molecules can not be further manipulated to another conformation. An attempt to move a "molecularly combed" molecule or its part, for example with assistance of AFM-tip (AFM - "Atomic Force Microscopy"), causes just cutting of polymer chain.

In WO 97/06278 a method of molecular combing of macromolecules by the action of a meniscus on highly specific surfaces is disclosed. Arrangement of macromolecules or displacement of parts of the chain of the macromolecules happens in solution. Another example of the method of "molecular combing" is known from U.S. 5,612,181. As in "molecular combing" a DNA molecule is first anchored by one end to a substrate in fluid while the main chain of the DNA molecule is dissolved in the fluid. Afterwards the stream of fluid is applied to stretch the DNA molecule along the stream. In an embodiment an external electrical field maybe applied to stretch the DNA molecule.

U.S. 5,314,829 relates to arranging DNA conformation in solution. According to the known method micro- or nano-machined grooves are first provided on a substrate surface. Solution of DNA molecule is loaded on this machined surface. The DNA molecule under gravity forces and/or an external electrical field applied perpendicular to the surface are dipping down to the linearly shaped bottom of the grooves. Final linear orientation of DNA molecule is due to mainly to orientation of mechanical walls of the grooves.

A special method was proposed, which is a combination of microscopic "modified molecular combing" and microscopic "molecular cutting" (see Hu, J. et al: Artifical DNA Patterns by Mechanical Nanomanipulation, vol. 2, no. 1 (January) 2002, pages 55-57). DNA strands are first aligned on a solid substrate to form a matrix of 2D networks. Atomic force microscopy is then used to cut the DNA network in order to fabricate fairly complex artificial patterns. Curved and wavy structures are constituted by a manipulation process based on the elastic behavior of DNA strands.

U.S. 5,677,126 discloses a specific surface prepared for biologial reactions. Using the surface made it possible to orient molecules after their attachment by at least one point by passage of the air/water meniscus.

U.S. 5,079,169 refers to a method and apparatus for manipulating a microscopic particle by single-beam gradient optical trapping.

Thus, it is the object of present invention to overcome the drawbacks of the methods of prior art and provide an improved method for arranging a polymer molecule on a support in such a manner that the polymer molecule can be manipulated to and can be fixed in arbitrary conformations and positions on the support surface.

According to the invention a method for arranging a polymer molecule such as a biomolecule on a support is provided according to claim 1.

A further aspect of the invention are the uses of the method according to the claims 23 to 25.

Different to all methods known from prior art, the inventive method allows adjustment of arbitrary conformations of a polymer molecule on a surface, not only straight linear alignment, including, for example, proper arrangement of branched or/and circular polymers like circular DNA. The surface layer provides optimized molecular interaction between the polymer molecule and the support. Compared to the known method of molecular combing, there is no need in moving meniscus for alignment of polymer molecule. Even single polymer molecule may be arranged in predefined conformations.

It is a further advantage of the method according to the invention that not only adjustment of the conformation of single polymeric molecules can be achieved, but also exact positioning of one individual polymer molecule with respect to another molecule of the same or different kind which is also situated on the surface layer may be performed. To achieve exact conformation and position of each molecule, situated on the support alone or as molecular assembly, is especially useful for the whole area of molecular- and nano-devices.

In addition, the defined adjustment of the molecular interaction between the polymer molecule and the support by means of the surface layer allows to (over)stretch the polymer molecule and to fix it in a stretched conformation which is of interest for investigating such polymers like ds-DNA.

It is well recognised that conventional lithography-based technology for production of computer chips is fast approaching the limits of its capabilities. Molecular electronics-based computation has attracted attention because it addresses the ultimate in dimensionally scaled systems: the ultradense and molecular scale. Polymer molecules that could replace parts of computer chips are known for some time, but it is still an open question how to place them on a chip in order to make working electrical circuits. The inventive method for controllable arranging polymer molecules on surfaces gives such opportunity.

Exemplary embodiments of the invention are described in detail in the following description in relation to accompanying drawings. In the figures:

| | |
|---|---|
| Figure 1 | shows a schematic representation of a support with a polymer molecule; |
| Figure 2 | shows a schematic representation for description of a conformation change of a polymer molecule; |
| Figure 3 | shows a schematic representation for description of stretching or over-stretching a polymer molecule; |
| Figure 4 | shows an example for manipulation of DNA molecules; |
| Figure 5 | shows orientation of DNA on the axes of surface layer composed from $CH_3(CH_2)_{17}NH_2$ |
| Figure 6 | shows orientation of poly-(allylamine)hydrochloride (positively charged poly-electrolyte); |
| Figure 7 | shows a schematic representation for description of different embodiments of pre-orientation of a polymer molecule on a 2dim-crystallized surface layer; |
| Figure 8 | shows orientation with simultaneous assembling of polystyrenesulphonate sodium salt (PSS); |
| Figures 9A and 9B | show manipulation of adsorbed polystyrenesulphonate sodium salt with assistance of water treatment; |
| Figure 10 | shows an example for not altering a surface layer with temperature at 40°C and 50°C, respectively; and |
| Figure 11 | shows the example for altering a surface layer (cf. Figure 10) with temperature at 60°C. |

For further understanding of the invention it will be useful to provide some additional definitions and explanations.

The terms "polymer" or "polymer molecule" as used here correspond to a special class of organic compounds which posses unique "polymeric" features. For example, being one integrated big molecule polymers behave in many tests as a set of independent particles where each particle corresponds to a piece of the polymeric chain with a certain length, which depends on the test method. Such pieces are named "thermodynamic segment", "mechanical segment", "persistent length" etc.. To be considered as a polymer the length of a molecule (or free path length between branching or cross-linking points) shall contain at least the length of such segment. Besides of first order temperature transitions like melting polymers exhibit many additional specific bulk transitions and states like a glass transition, $\alpha$-, $\beta$- and $\gamma$-transitions (e.g. in polyethylene), elastic state etc. Further details can be found in: P.J. Flory, "Principles of Polymer Chemistry", 16th ed., Cornell University Press, N.Y., 1995.

Polymers within the scope of the present application include all known classes of synthetic and natural ("biomolecules") polymers, including polyolefines, polyamides, polyesters, polyethers, silicones, polysilanes, any kind of polyelectrolytes, ionic polymers (where the main chain is composed from bivalent ions), ss- and ds-DNA, the various proteins, lipoproteins, polysaccharides etc. Polymers comprise also any kind of co-polymers. A polymer can be in form of a complex with another polymer, such as a polyelectrolyte complex, or with low or middle molecular weight organic or inorganic substances or ions. A polymer can be used as a one kind polymer or as a complex or as any desired combination thereof.

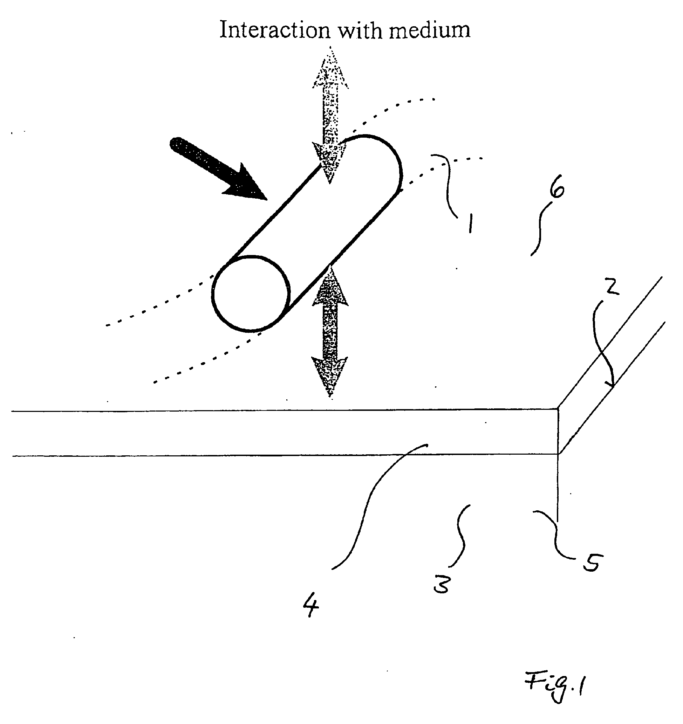

Now turning to Figure 1, a polymer molecule 1 is placed on a support 5 provided by a substrate 3 and a surface layer 4. The polymer molecule 1 interacts with the support 5 and a medium 6 surrounding the polymer molecule 1. If interaction with the support ($I_S$) 5 is stronger than interaction with the medium ($I_M$) 6 i.e when:

$$I_S > I_m \qquad\qquad (1)$$

then the polymer molecule 1 is considered to be placed (situated) on the support 5. If situation is reversed, i.e.:

$$I_S < I_m \qquad\qquad (2)$$

then the polymer molecule 1 leaves from the support 5 to the medium 6 (e.g. dissolved) and it is not considered anymore as placed (situated) on the support 5, even if one end of polymer chain is anchored to the support.

The support 5 comprising the substrate 3 and the surface layer 4 may be any material whose cohesion and chemical stability are sufficient to withstand the conditions of the method according to invention. The support 5 may consist of an organic or inorganic substance such as organic or inorganic polymer, metal, metal oxide, sulfide or salt with organic or inorganic acid, semiconductor element or an oxide of semiconductor element, optical element or combination thereof such as glass or ceramic. Examples particularly comprise glass, quartz, surface oxidized silicon, graphite (including "Highly Oriented Pyrolytic Graphite" - HOPG), mica and molybdenum sulfide. As support 5, there may be used flat supports such as slides, especially atomically flat supports, but also beads, particles, bars, fibers or a structured support.

The surface layer 4 having a certain thickness (depth) is a surface molecular or atomic upper layer of the support 5 which is physico-chemically different from a volume part of the support 5 namely the substrate 3. The surface layer 4 can be present just as the upper layer of the substrate 3, with or without special chemical, physico-chemical or plasma-chemical modification of the surface 2. As the simplest case one can provide a substrate such as freshly cleaved HOPG or mica and the upper atomic layer (which itself differs from the underlying structure) of this substrate immediately develops a surface layer through the adsorption of the components of surrounding medium (e.g. gas molecules from the atmosphere). In case of most of the industrial polymers the surface layer 4 occurs at production step from the melt or hot solutions through the oxidation of the surface 2 by atmospheric oxygen.

The surface layer 4 can be specially constructed through the chemical modification of the substrate surface 2 (introduction of new functional groups) by conventional chemical reactions or by special methods like plasma-chemical modification. In this case the surface layer 4 is an inherent part of the support 5 integrated to the substrate 3 (support volume) through valence bonds.

On the other hand, the surface layer 4 can comprise of any adsorbed mono- or multimolecular layer which is bound to the substrate 3 by physical forces (like London or van-der-Waals forces), by any kind of charge interaction (like Coloumb forces, dipole-dipole interactions), by other interactions like hydrogen bonds or by any combination of such binding forces. Accordingly, in the scope of the present invention the surface layer 4 can be formed by any desired kind of coating which includes, but which is not limited to casted coatings, spin-coatings, vacuum-evaporated and plasma-deposited coatings, organized molecular layers like Langmuir-Blodgett layers, polyelectrolyte complexes and polyelectrolyte multi-layers made by Layer-by-Layer assembly technique, two-dimensional (2D)-crystallized layers composed from low-, middle- or high-molecular (including polymers) weight substances. During molecular arranging (manipulating) according to the method of the present invention the surface layer 4 can stay unchanged or it can change, e.g. it can change the surface charge, hydrophobic-hydrophilic balance or it can move together with manipulated polymer under external force.

The surface layer 4 can have certain zones (areas) or directions (axes) - "Sites" of preferential adsorption with respect to the polymer to be arranged. At these Sites the Is is sufficiently different from the rest of the surface, thus the polymer initially adsorbed on the surface can have already certain orientation which is due to the external force developed by said sites and which influences also the further molecular arranging processes. Such sites are comprising, but not limited to surface defects such as grooves, networks, borders between crystalline domains etc., occurring naturally or artificially in the surface layer. In a preferred embodiment the surface layer has a 2D-cristallized structure, especially composed from amphiphilic molecules, where said sites comprise linear lamellar directions (axes) and borders between neighboring 2D-crystalline domains.

In case the polymer molecule 1 (cf. Figure 1) is responsive to electric or magnetic fields, then to adjust the interaction between the polymer molecule 1 and the support 5 at stage of molecular dislocation, a magnetic or an electric field can be used which acts perpendicularly (or at certain angle) to the surface 2 to reduce the binding force between the polymer molecule 1 and the support 5 and to enhance the molecular mobility up to a level when dislocating across the support 5 becomes possible. For instance, the field can orient molecular parts in the polymer molecule 1 or the surface layer 4, which influences the interaction between the two.

One can enhance the molecular mobility and allow dislocating of a polymer molecule on the support not only by application of external fields, but also by excitation of the polymer, the support, or a complex of the polymer with the surface layer by light. At properly chosen excitation conditions the conformation and the position of the excited molecule will be still fixed on the surface but a dislocation of the molecule or its part under external force will be possible without polymer chain breakage. For instance, the light can reduce the glass transition temperature of the surface layer, thereby changing the interaction between the polymer molecule and the support.

In case the aim of manipulation is to stretch or over-stretch the polymer molecule 1 it could be useful to anchor at least one end of the polymer 1 to the support 5 to prevent the movement of the polymer molecule 1 as a whole under external field. For example, to assay the base pairs sequence of DNA it could be very useful to stretch and over-stretch the polymer molecule 1 to make each base pair more available for analysis. Another possible task of anchoring is reliable fixation of different polymer molecules with respect to each other, to avoid displacing of the molecular position under manipulation or under the change of surrounding conditions. Such task is especially important for molecular arrays and molecular chips.

As a support 5 to which the polymer molecule 1 is anchored one can use also particles, fibers and other objects. For example, if one uses electric or magnetic field or optical tweezers to develop external force and to approach proper placing of the polymer molecule 1 or to stretch it, in case when the polymer molecule 1 itself is not sensitive enough to such field, then it could be useful to link the polymer molecule to an object which is sensitive to the field (e.g. to an iron nano- or micro-particle).

Single molecule force spectroscopy on polysaccharides using a force microscopy set-up revealed that a single polymer can withstand forces between 1,5 and 2 nN before breaking (cf.

M. Rief et al., Science 275 (1997) 1295). The force required to manipulate a polymer across a surface should therefore be smaller in order to avoid breakage during the manipulation.

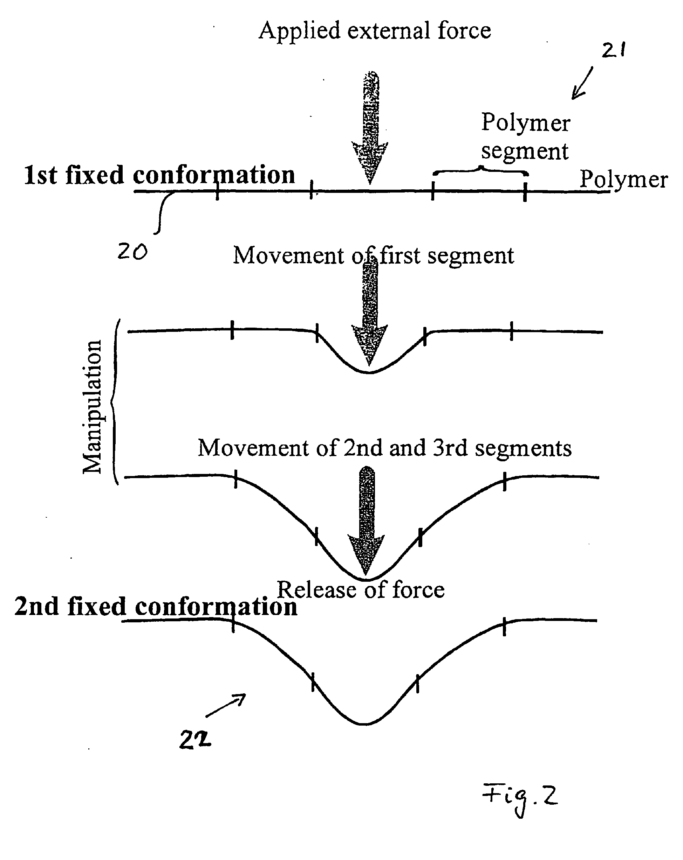

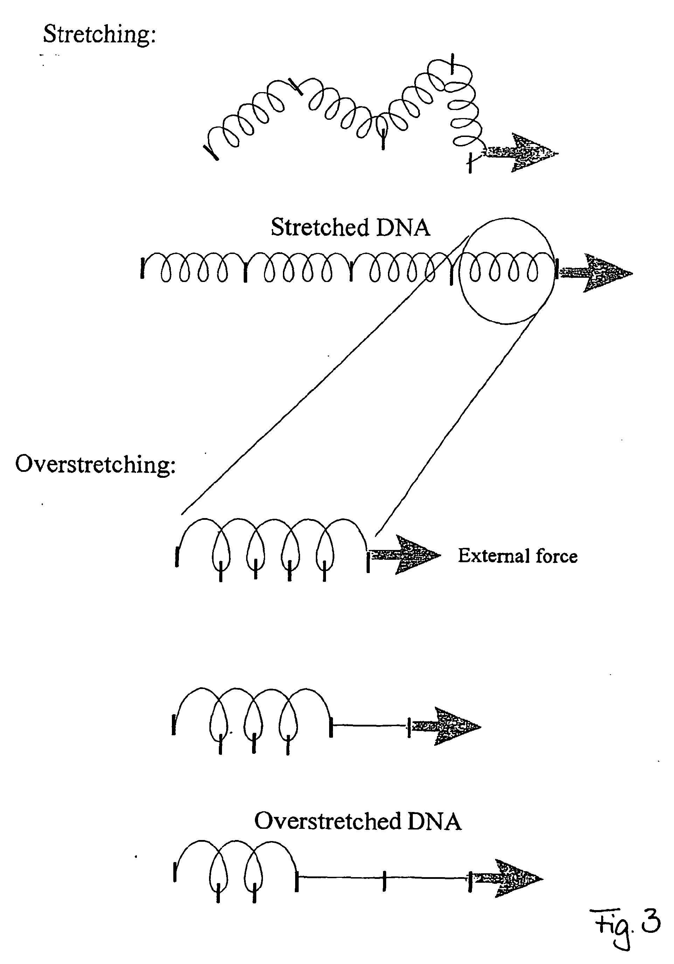

Referring now to figures 2 and 3, in an extreme case the geometry of binding sites, the adsorption process and the interaction of the polymer molecule with said sites (attractive forces or "Force frame" developed by site with respect to polymer) can be organized very perfectly with achievement of desired configuration and position of polymer already at step of initial polymer adsorption onto surface layer. In this case only very minor corrections/arrangements of initial configuration/position will be necessary to be performed, if any. After step of placing the polymer molecule on such surface layer one can change a $1^{st}$ conformation to a $2^{nd}$ conformation (e.g. with higher orientation degree and longer pieces of polymer chain stretched on the sites). Changing a polymer chain 20 from a $1^{st}$ conformation 21 to a $2^{nd}$ conformation 22 is schematically depicted in Figure 2.

Sometimes just an increased temperature can be used, or the system is just kept a certain while under a specific medium which decreases Is (or increases $I_M$) and allows polymer chain to approach new conformation in the field of force developed by sites. In this case no special external force is required. If already $1^{st}$ observed conformation meets the requirements of given application, it means that molecules are already properly arranged during adsorption process under the forces developed by sites and no further operation is requested. In this case the steps of achievement of $1^{st}$ and $2^{nd}$ conformations and molecular arrangement proceed simultaneously in one step.

The term external force as used in the present application is any external (with respect to the polymer molecule 1 in Figure 1) force applied to the polymer molecule to be arranged on the support 5. The external force can be applied perpendicular or at certain angle with respect to the main polymer chain 20 (cf. Figure 2) or axial, i.e. parallel to the main polymer chain (cf. Figure 3). In the last case the polymer will be stretched and over-stretched (if polymer chain has helical (coiled), double helical or Zig-Zag or analogous conformation, see Figure 3. An external force can be applied directly to the polymer chain or through any substrate like particle, fiber etc., to which the polymer is anchored. External force may be attractive force developed by "Sites" mentioned above.

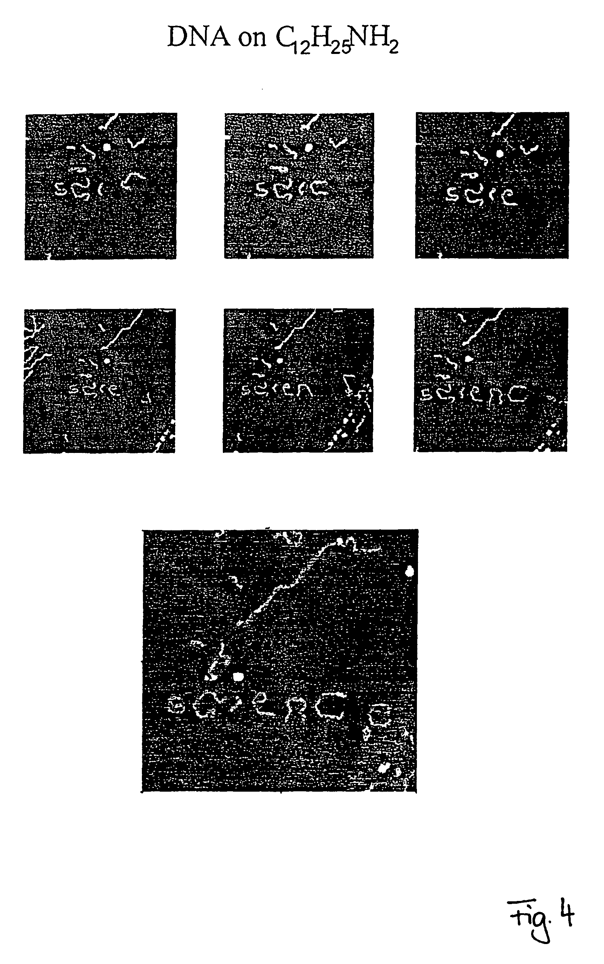

Referring to Figures 4 to 11, examples of the method according to the invention. Figure 4 shows the result of manipulating and positioning a DNA chain on the support surface (writing word "Science" on the surface by DNA-molecules). Details of the method performed are as follows. Chloroform solution of $CH_3(CH_2)_{11}NH_2$ at concentration of $3 \times 10^{-2}$ g/l is spin coated (40 rps) on freshly cleaved graphite surface and dried at 35° C for 10 minutes in air. DNA (DNA set: Step-Ladder 1018 produced by Mo Bi Tec GmbH, Germany) was diluted by water (purified by millipore Milli-Q Sythesis A10 system) to concentration $10^{-3}$ g/l and diluted DNA solution was deposited on the graphite surface for period from 5 to 30 seconds and removed by bringing sample in rotation (40 rps). Alternatively applied DNA solution could be blown away with compressed gas (nitrogen) or shaken out. The single DNA molecules on the surface were imaged and manipulated with Scanning Force Microscopy (SFM) tip, (Nanoscope IIIa, Digital Instruments, USA), an E-scanner in a range of scan lengths from 5 $\mu$m to 0.3 $\mu$m, and commercial Si cantilevers (length 125 $\mu$m and width 30 $\mu$m) with spring constants between 17 and 64 Nm$^{-1}$ were used. Imaging is performed in tapping mode, manipulation is performed by bringing tip in contact with sample and moving the tip in desired direction (best analogue is the manipulation of a rope which lies free on a table by vertical pen). This example shows features of the method according to invention, namely ability to precisely manipulate individual polymer molecule to any desired conformation (shape) and to arrange exact position of several molecules on the surface.

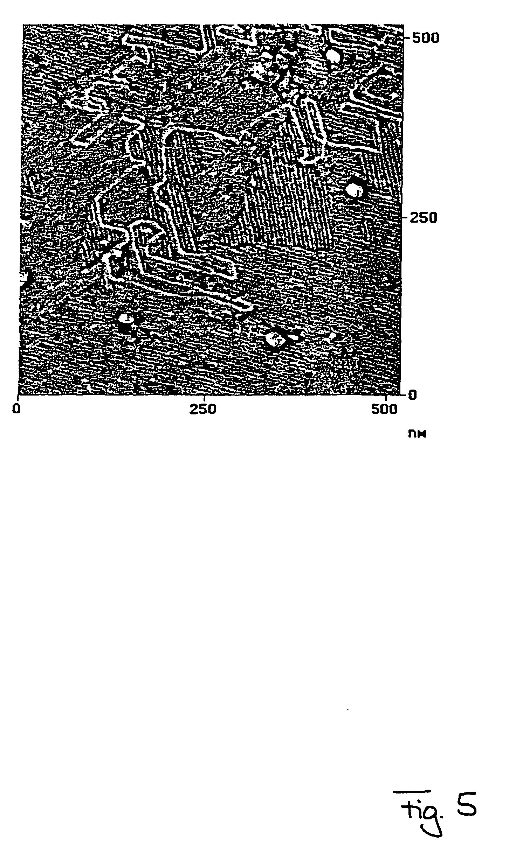

Figure 5 shows orientation of DNA on the axes of surface layer composed from $CH_3(CH_2)_{17}NH_2$. In this case the method described in relation to Figure 4 is repeated except $CH_3(CH_2)_{11}NH_2$ is replaced by $CH_3(CH_2)_{17}NH_2$. DNA is oriented spontaneously during adsorption on the surface layer with appearance of a few hundred nanometers long stretched DNA parts.

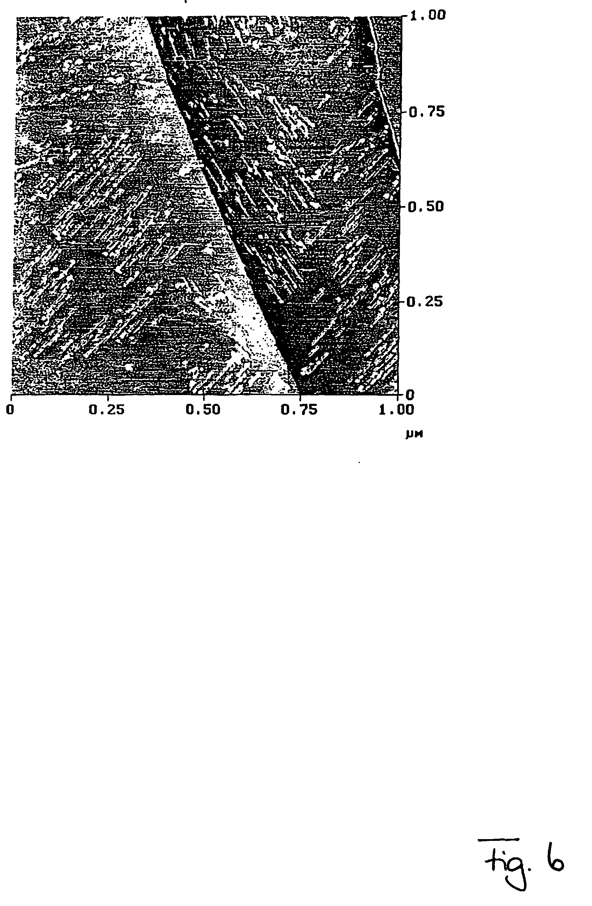

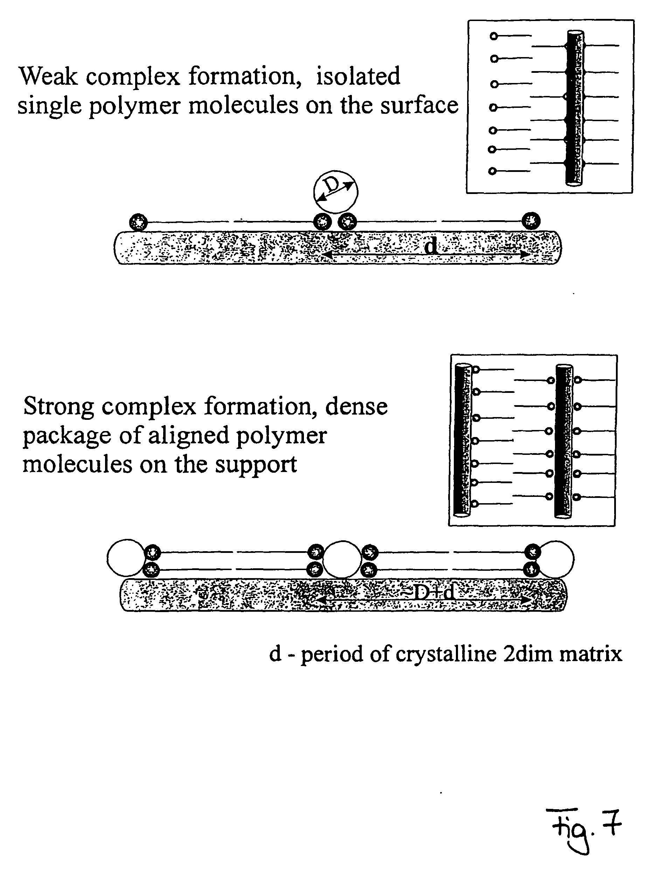

Figure 6 shows orientation of poly-(allylamine)hydrochloride (positively charged poly-electolyte). On the axes of surface layer composed from $CH_3(CH_2)_{17}COOH$ the method described in relation to Figure 4 is repeated where the DNA is replaced by poly-(allylamine)hydrochloride, the $CH_3(CH_2)_{11}NH2$ is replaced by $CH_3(CH_2)_{17}COOH$ and polymer solution has concentration $10^{-3}$ g/l. An example of "weak" complex formation between polymer and surface layer, i.e. the polymer molecule perturbs the surface layer only slightly without changing its integrity, in lattice paramters etc., with appearance of single isolated polymer molecules which are oriented with stretching is schematically illustrated in Figure 7 (upper part).

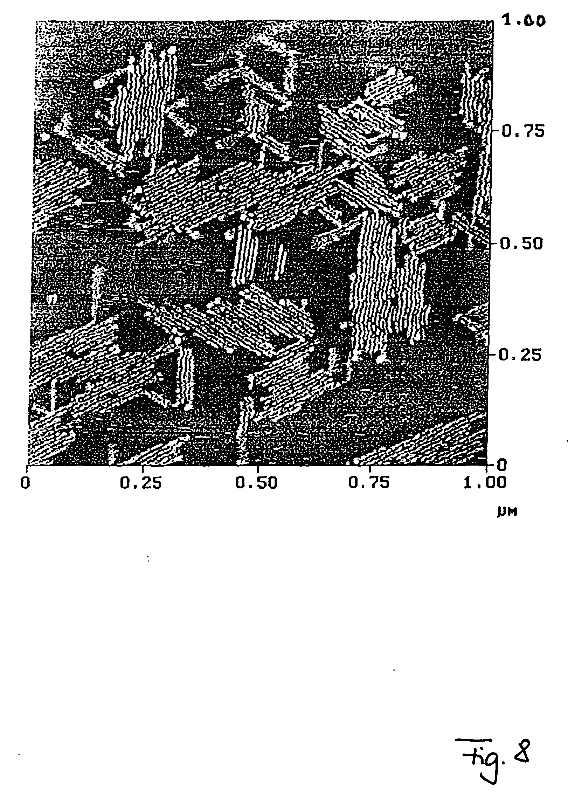

Orientation with simultaneous assembling of polystyrenesulphonate sodium salt (PSS) (positively charged polyelectrolyte) is shown in Figure 8. The method described in relation to Figure 5 is repeated where the DNA is replaced by PSS except drying for 10 min at 35° is excluded. The example depicted in Figure 8 illustrates "strong" complex formation with appearance of dense assemblies of oriented and stretched polymer molecules. This situation is schematically illustrated in Figure 7 (lower part).

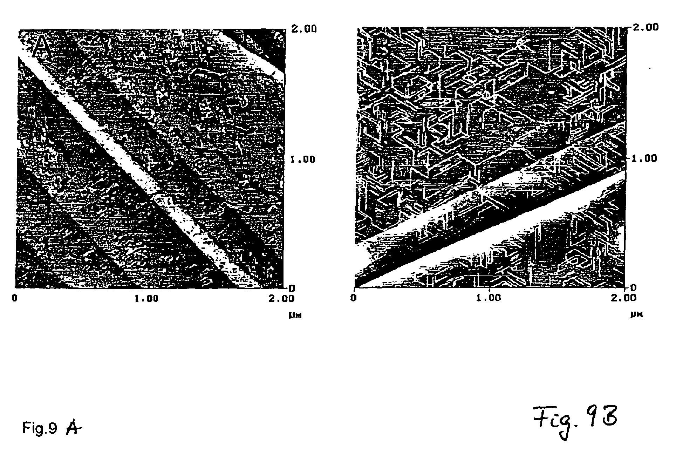

Figures 9A and 9B show manipulation of adsorbed polystyrenesulphonate sodium salt (PSS) with assistance of water treatment. In Figure 9A, the method described above in relation to Figure 8 is reproduced except of additional intermediate drying of surface layer composed from $CH_3(CH_2)_{17}NH_2$ for 10 min at 35°C. To receive the result shown in Figure 9 the sample depicted in Fig. 9A was treated by water for 5 min. The examples in Figure 9A and 9B illustrate the opportunity to perform manipulation of polymer molecules by change of surrounding medium (i.e. through the adjustment of $I_S$ and $I_m$ ) in the field of forces developed by special zones of structured surface layer.

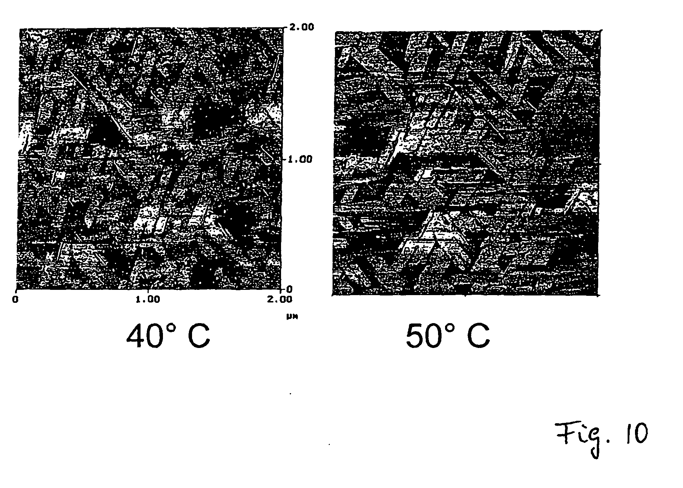

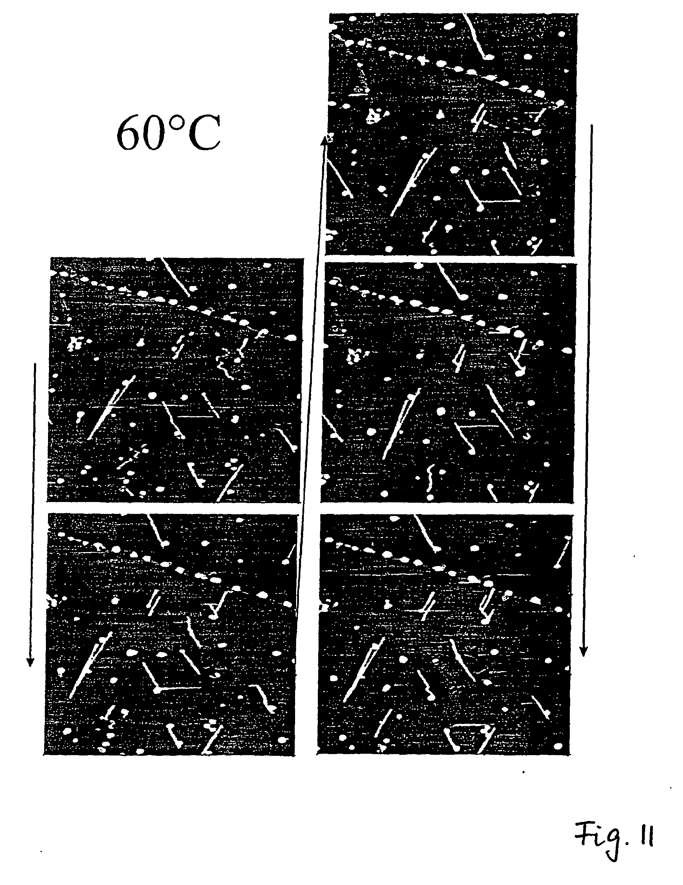

Figure 10 shows an example for altering a surface layer (amphiphilic molecules on graphite) with temperature. Below ~55°C the surface layer is crystalline and keeps applied polymer molecules immobilised. At ~55°C the surface layer melts, thereby allowing the polymer molecules to diffuse. At 60°C a series of 5 images has been recorded with a scanning force microscope, demonstrating that the two marked polymer molecules diffuse across the surface (see Figure 11).

The features disclosed in this specification and/or the claims may be material for the realization of the invention in its various embodiments, taken in isolation or in various combinations thereof.

## Claims

1. A method for arranging a polymer molecule such as a biomolecule on a support, the method comprising the following steps:

> - providing a substrate (3) having a surface (2);
> - providing a surface layer (4) on said surface (2) of the substrate (3), said substrate (3) and said surface layer (4) providing a support (5);
- placing a polymer molecule (1) on said surface layer (4) in a first position; and
- adsorbing the polymer molecule (1) on said surface layer (4) by interaction with binding sites of preferential adsorption, thereby, providing an adsorbed state of the polymer molecule (1), the polymer molecule (1) having a first conformation on said surface layer (4);

wherein said surface layer (4) is configured to adjust predefined molecular interaction between the polymer molecule (1) and said support (5) to allow fixing of the first conformation of said polymer molecule (1), and in said adsorbed state of the polymer molecule (1) dislocating the polymer molecule (1) across said surface layer (4) relative to said support (5) by an external force wherein the step of dislocating comprises steps of dislocating the polymer molecule (1) in said adsorbed state across said surface layer (4) by changing said first position of the polymer molecule (1) to a second position different from the first position on the surface layer (4), and fixing the polymer molecule (1) on said surface layer (4) in said second position by means of said predefined molecular interaction between the polymer molecule (1) and said support (5).

2. The method according to claim 1, wherein the method comprises a step for subsequently fixing the polymer molecule (1) on the surface layer (4).

3. The method according to claim 1 or 2, wherein the method comprises a step of dislocating in said adsorbed state the polymer molecule (1) across said surface layer (4) by manipulation of said first conformation of the polymer molecule (1) to a second conformation different from the first conformation of the polymer molecule (1), and fixing the polymer molecule (1) on the surface layer (4) in said second conformation by means of said molecular interaction between the polymer molecule (1) and said support (5).

4. The method according to one of the preceding claims, the method further comprising a step of dislocating the polymer molecule (1) across the surface layer (4) by applying a force which is smaller than about 2nN.

5. The method according to one of the preceding claims, wherein the step of providing said surface layer (4) on said surface (2) of said substrate (3) comprises a step of forming domains and/or axes and/or further binding sites in said surface layer (4).

6. The method according to claim 5, wherein said external force comprises an attractive force provided at least partly by said domains and/or axes and/or further binding sites in said surface layer (4).

7. The method according to one of the preceding claims, wherein said surface layer (4) is self assembling.

8. The method according to one of the preceding claims, wherein said step for providing said surface layer (4) on said surface of said substrate (5) comprises a step for using one or more of the following methods:

- a chemical method with appearance of new chemical functionalities covalently bound to said surface (2) of said substrate (3);
- plasma-chemical method;
- thin or ultra-thin coating applied by surface adsorption method;
- thin or ultra-thin spin-coating;
- thin or ultra-thin coating applied by vacuum deposition method;
- a Langmuir-Blodgett technique or a self-organized film technology;
- Layer-by-Layer polyelectrolyte assembling; and
- 2D-crystallization of low-, middle- or high molecular weight substances or their complexes on the surface.

9. The method according to one of the preceding claims, wherein the method further comprises a step for altering said predefined molecular interaction between the polymer molecule (1) and said support (5).

10. The method according to claim 9, wherein said step for altering said predefined molecular interaction comprises a step for placing said surface layer (4) with the polymer molecule (1) provided thereon into a liquid medium.

11. The method according to claim 9 or 10, wherein said step for altering said predefined molecular interaction comprises a step for drying said surface layer (4) with the polymer molecule (1) provided thereon.

12. The method according to one of the claims 9 to 11, wherein said step for altering said predefined molecular interaction comprises a step for changing a temperature of said surface layer (4).

13. The method according to one of the claims 9 to 12, wherein said step for altering said predefined molecular interaction comprises a step for applying an electric or/and magnetic field oriented perpendicular or at certain angle with respect to said surface of said support (5).

14. The method according to one of the claims 9 to 13, wherein said step for altering said predefined molecular interaction comprises a step for exciting the polymer by light.

15. The method according to one of the preceding claims, wherein said external force is provided by using one of the following fields: electrical filed, magnetic field, optical field and mechanical field, or any combination thereof.

16. The method according to one of the preceding claims, wherein a scanning probe microscope (SPM) is used for applying said external force.

17. The method according to one of the preceding claims, wherein the polymer molecule (1) comprises a polynucleotide such as DNA or RNA, a polypeptide such as protein, an antibody or antigen-antibody system, a polysaccharide, or a desired mixture of biomolecules.

18. The method according to one of the preceding claims, wherein said surface layer (4) comprises an inorganic polymer, an organic polymer, a metal, an organic low molecular substance, a metal oxide, a sulfide, a semiconductor, or an optical element, or a any combination thereof.

19. The method according to one of the preceding claims, wherein said substrate (5) is atomically flat.

20. The method according to one of the preceding claims, wherein said substrate (5) comprises glass, surface oxidized silicon, gold, molybdenum sulfide, highly oriented pyrolitic graphite (HOPG) or mica.

21. The method according to one of the preceding claims, wherein the method comprises a step for anchoring at least one end of the polymer molecule (1) to said support (5).

22. The method according to one of the preceding claims, wherein the method comprises a step for anchoring at least one end of the polymer molecule (1) to be arranged to a fiber, a micro-particle or a nano-particle.

23. Use of a method according to one of the claims 1 to 22 for recognition, detecting or analysis of a component of surrounding medium or/and of a polymer molecule (1) to be arranged.

24. Use of a method according to one of the claims 1 to 22 for recognition, detecting or analyzing of a molecule or chemical groups of a surface layer (4).

25. Use of a method according to one of the claims 1 to 22 for constructing a molecular device.

**Patentansprüche**

1. Verfahren zum Anordnen eines Polymermoleküls wie zum Beispiel einem Biomolekül auf einem Träger, wobei das Verfahren die folgenden Schritte umfasst:

   - Bereitstellen eines Substrats (3) mit einer Oberfläche (2);
   - Bereitstellen einer Oberflächenschicht (4) auf der Oberfläche (2) des Substrats (3), wobei das Substrat (3) und die Oberflächenschicht (4) einen Träger (5) bereitstellen;
   - Platzieren eines Polymermoleküls (1) auf der Oberflächenschicht (4) in einer ersten Position; und
   - Adsorbieren des Polymermoleküls (1) auf dieser Oberflächenschicht (4) mittels Wechselwirkung mit Bindungsstellen bevorzugter Adsorption, wodurch ein adsorbierter Zustand des Polymermoleküls (1) gebildet wird, wobei das Polymermolekül (1) eine erste Konformation auf der Oberflächenschicht (4) aufweist;

   wobei die Oberflächenschicht (4) konfiguriert ist, eine vordefinierte molekulare Wechselwirkung zwischen dem Polymermolekül (1) und dem Träger (5) einzustellen, um eine Fixierung der ersten Konformation des Polymermoleküls (1) und in dem adsorbierten Zustand des Polymermoleküls (1) eine Verlagerung des Polymermoleküls (1) über die Oberflächenschicht (4) relativ zum Träger (5) durch eine äußere Kraft zu ermöglichen, und wobei der Schritt zum Verlagern die Schritte umfasst, das Polymermolekül (1) im adsorbierten Zustand mittels Verändern der ersten Position des Polymermoleküls (1) in eine zweite Position über die Oberflächenschicht (4) zu verlagern, die sich von der ersten Position auf der Oberflächenschicht (4) unterscheidet, und das Polymermolekül (1) auf der Oberflächenschicht (4) in der zweiten Position mittels der vordefinierten molekularen Wechselwirkung zwischen dem Polymermolekül (1) und dem Träger (5) zu fixieren.

2. Verfahren nach Anspruch 1, wobei das Verfahren einen Schritt umfasst, das Polymermolekül (1) auf der Oberflächenschicht (4) nachträglich zu fixieren.

3. Verfahren nach Anspruch 1 oder 2, wobei das Verfahren einen Schritt umfasst, das Polymermolekül (1) in dem adsorbierten Zustand mittels Manipulation der ersten Konformation des Polymermoleküls (1) zu einer zweiten Konformation über die Oberflächenschicht (4) zu verlagern, die sich von der ersten Konformation des Polymermoleküls (1) unterscheidet, und das Polymermolekül (1) auf der Oberflächenschicht (4) in der zweiten Konformation mittels der molekularen Wechselwirkung zwischen dem Polymermolekül (1) und dem Träger (5) zu fixieren.

4. Verfahren nach einem der vorhergehenden Ansprüche, wobei das Verfahren weiterhin einen Schritt umfasst, das Polymermolekül (1) über die Oberflächenschicht (4) mittels Anlegen einer Kraft zu verlagern, die kleiner als etwa 2 nN ist.

5. Verfahren nach einem der vorhergehenden Ansprüche, wobei der Schritt des Bereitstellens der Oberflächenschicht (4) auf der Oberfläche (2) des Substrats (3) einen Schritt umfasst, Domänen und/oder Achsen und/oder weitere Bindungsstellen in der Oberflächenschicht (4) zu bilden.

6. Verfahren nach Anspruch 5, wobei die äußere Kraft eine anziehende Kraft umfasst, die mindestens teilweise mittels der Domänen und/oder der Achsen und/oder der weiteren Bindungsstellen in der Oberflächenschicht (4) bereitgestellt ist.

7. Verfahren nach einem der vorhergehenden Ansprüche, wobei die Oberflächenschicht (4) selbstanordnend ist.

8. Verfahren nach einem der vorhergehenden Ansprüche, wobei der Schritt des Bereitstellens der Oberflächenschicht (4) auf der Oberfläche des Substrats (5) einen Schritt zur Verwendung eines oder mehrerer der folgenden Verfahren umfasst:

   - ein chemisches Verfahren mit dem Auftreten von neuen chemischen Funktionalitäten, die kovalent an die Oberfläche (2) des Substrats (3) gebunden sind;
   - ein plasma-chemisches Verfahren;
   - eine dünne oder ultradünne Beschichtung, die mittels Oberflächenadsorptionsverfahren aufgebracht wird;
   - ein dünnes oder ultradünnes Schleuderbeschichten;
   - eine dünne oder ultradünne Beschichtung, die mittels Vakuum-Abscheidungsverfahren aufgebracht wird;
   - eine Langmuir-Blodgett-Technik oder eine selbstorganisierende Filmtechnologie;
   - schichtweise polyelektrolytische Anordnung; und
   - 2D-Kristallisierung von Substanzen mit geringem, mittlerem oder hohem Molekulargewicht oder deren Komplexe auf der Oberfläche.

9. Verfahren nach einem der vorhergehenden Ansprüche, wobei das Verfahren weiterhin einen Schritt umfasst, die vordefinierte molekulare Wechselwirkung zwischen dem Polymermolekül (1) und dem Träger (5) zu verändern.

10. Verfahren nach Anspruch 9, wobei der Schritt zum Verändern der vordefinierten molekularen Wechselwirkung einen Schritt umfasst, die Oberflächenschicht (4) mit dem darauf bereitgestellten Polymermolekül (1) in einem flüssigen Medium anzuordnen.

11. Verfahren nach Anspruch 9 oder 10, wobei der Schritt zum Verändern der vordefinierten molekularen Wechselwirkung einen Schritt umfasst, um die Oberflächenschicht (4) mit dem darauf bereitgestellten Polymermolekül (1) zu trocknen.

**12.** Verfahren nach einem der Ansprüche 9 bis 11, wobei der Schritt zum Verändern der vordefinierten molekularen Wechselwirkung einen Schritt umfasst, um eine Temperatur der Oberflächenschicht (4) zu verändern.

**13.** Verfahren nach einem der Ansprüche 9 bis 12, wobei der Schritt zum Verändern der vordefinierten molekularen Wechselwirkung einen Schritt umfasst, ein elektrisches und/oder magnetisches Feld anzulegen, das senkrecht oder in einem bestimmten Winkel bezogen auf die Oberfläche des Trägers (5) orientiert ist.

**14.** Verfahren nach einem der Ansprüche 9 bis 13, wobei der Schritt zum Verändern der vordefinierten molekularen Wechselwirkung einen Schritt umfasst, das Polymer mittels Licht anzuregen.

**15.** Verfahren nach einem der vorhergehenden Ansprüche, wobei die äußere Kraft unter Verwendung eines der folgenden Felder bereitgestellt wird: elektrisches Feld, magnetisches Feld, optisches Feld und mechanisches Feld, oder eine beliebige Kombination hieraus.

**16.** Verfahren nach einem der vorhergehenden Ansprüche, wobei ein Rastersondenmikroskop (SPM) zum Anlegen der äußeren Kraft verwendet wird.

**17.** Verfahren nach einem der vorhergehenden Ansprüche, wobei das Polymermolekül (1) ein Polynucleotid wie zum Beispiel DNA oder RNA, ein Polypeptid wie zum Beispiel ein Protein, ein Antikörper oder Antigen-Antikörper-System, ein Polysaccharid oder eine gewünschte Mischung aus Biomolekülen umfasst.

**18.** Verfahren nach einem der vorhergehenden Ansprüche, wobei die Oberflächenschicht (4) ein anorganisches Polymer, ein organisches Polymer, ein Metall, eine organische Substanz mit geringem Molekulargewicht, ein Metalloxid, ein Sulfid, einen Halbleiter oder ein optisches Element oder eine beliebige Kombination hieraus umfasst.

**19.** Verfahren nach einem der vorhergehenden Ansprüche, wobei das Substrat (5) atomar eben ist.

**20.** Verfahren nach einem der vorhergehenden Ansprüche, wobei das Substrat (5) Glas, Silicium mit oxidierter Oberfläche, Gold, Molybdänsulfid, hochorientiertes pyrolytisches Graphit (HOPG) oder Glimmer umfasst.

**21.** Verfahren nach einem der vorhergehenden Ansprüche, wobei das Verfahren einen Schritt umfasst, mindestens ein Ende des Polymermoleküls (1) an dem Träger (5) zu verankern.

**22.** Verfahren nach einem der vorhergehenden Ansprüche, wobei das Verfahren einen Schritt umfasst, mindestens ein Ende des Polymermoleküls (1) zu verankern, um an einer Faser, einem Mikropartikel oder einem Nanopartikel angeordnet zu werden.

**23.** Verwendung eines Verfahrens nach einem der Ansprüche 1 bis 22 zum Erkennen, Detektieren oder Analysieren eines Bestandteils eines umgebenden Mediums und/oder eines anzuordnenden Polymermoleküls (1).

**24.** Verwendung eines Verfahrens nach einem der Ansprüche 1 bis 22 zum Erkennen, Detektieren oder Analysieren eines Moleküls oder von chemischen Gruppen auf einer Oberflächenschicht (4).

**25.** Verwendung eines Verfahrens nach einem der Ansprüche 1 bis 22 zum Bau einer molekularen Vorrichtung.

**Revendications**

**1.** Méthode pour l'arrangement d'une molécule polymère comme une biomolécule sur un support, la méthode comprenant les étapes suivantes :

- mise à disposition d'un substrat (2) présentant une surface (2) ;
- mise à disposition d'une couche superficielle (4) sur ladite surface (2) du substrat (3), ledit substrat (3) et ladite couche superficielle (4) formant un support (5) ;
- placement d'une molécule polymère (1) sur ladite couche superficielle (4) à une première position ;
- adsorption de la molécule polymère (1) sur ladite couche superficielle (4) par interaction avec des sites liants d'adsorption préférentielle, réalisant ainsi un état adsorbé de la molécule polymère (1), la molécule polymère (1) présentant une première conformation sur ladite couche superficielle (4) ;

dans laquelle ladite couche superficielle (4) est configurée de manière à régler une interaction moléculaire prédéfinie entre la molécule polymère (1) et ledit support (5) afin de permettre la fixation de la première conformation de ladite molécule polymère (1), et, dans ledit état adsorbé de la molécule polymère (1), la dislocation de la molécule polymère (1) à travers ladite couche superficielle (4) par rapport audit support (5) par une force externe, dans laquelle l'étape de dislocation comprend des étapes de dislocation de la molécule polymère (1) dans ledit état adsorbé à travers ladite couche superficielle 84) en faisant passer ladite première position de la molécule polymère (1) à une deuxième position différente de la première position sur la couche superficielle (4), et fixation de la molécule polymère (1) sur ladite couche superficielle (4) à ladite deuxième position au moyen de ladite interaction moléculaire prédéfinie entre la molécule polymère (1) et ledit support (5).

2. Méthode selon la revendication 1, dans laquelle la méthode comprend une étape destinée à fixer par la suite la molécule polymère (1) sur la couche superficielle (4).

3. Méthode selon la revendication 1 ou 2, dans laquelle la méthode comprend une étape de dislocation dans ledit état adsorbé de la molécule polymère (1) à travers ladite couche superficielle (4) par manipulation de ladite première conformation de la molécule polymère (1) pour donner une deuxième conformation différente de la première conformation de la molécule polymère (1), et fixation de la molécule polymère (1) sur la couche superficielle (4) dans ladite deuxième conformation au moyen de ladite interaction moléculaire entre la molécule polymère (1) et ledit support (5).

4. Méthode selon l'une quelconque des revendications précédentes, la méthode comprenant en outre une étape de dislocation de la molécule polymère (1) à travers la surface superficielle (4) par application d'une force inférieure à environ 2 nN.

5. Méthode selon l'une quelconque des revendications précédentes, dans laquelle l'étape de réalisation de ladite couche superficielle (4) sur ladite surface (2) dudit substrat (3) comprend une étape de formation de domaines et/ou d'axes et/ou d'autres sites liants dans ladite couche superficielle (4).

6. Méthode selon la revendication 5, dans laquelle ladite force externe comprend une force attrayante fournie au moins partiellement par lesdits domaines et/ou axes et/ou autres sites liants dans ladite couche superficielle (4).

7. Méthode selon l'une quelconque des revendications précédentes, dans laquelle ladite couche superficielle (4) est auto-assemblante.

8. Méthode selon l'une quelconque des revendications précédentes, dans laquelle ladite étape de réalisation de ladite couche superficielle (4) sur ladite surface (2) dudit substrat (3) comprend une étape pour l'utilisation d'une ou plusieurs des méthodes suivantes :

- une méthode chimique avec apparition de nouvelles fonctionnalités chimiques liées par covalence à ladite surface (2) dudit substrat (3) ;
- une méthode plasma-chimique ;
- revêtement mince ou ultramince appliqué par la méthode d'adsorption en surface ;
- revêtement centrifuge mince ou ultramince ;
- revêtement mince ou ultramince appliqué par la méthode de dépôt sous vide ;
- une technique Langmuir-Blodgett ou une technologie de film auto-organisé ;
- assemblage de polyélectrolyte couche par couche ; et
- cristallisation en 2D de substances de masse moléculaire basse, moyenne ou élevée ou de leurs complexes à la surface.

9. Méthode selon l'une quelconque des revendications précédentes, dans laquelle la méthode comprend en outre une étape de modification de ladite interaction moléculaire prédéfinie entre la molécule polymère (1) et ledit support (5).

10. Méthode selon la revendication 9, dans laquelle ladite étape de modification de ladite interaction moléculaire prédéfinie comprend une étape pour le placement de ladite couche superficielle (4) portant la molécule polymère (1) dans un milieu liquide.

11. Méthode selon la revendication 9 ou 10, dans laquelle ladite étape de modification de ladite interaction moléculaire prédéfinie comprend une étape pour le séchage de ladite couche superficielle (4) portant la molécule polymère (1).

**12.** Méthode selon l'une quelconque des revendications 9 à 11, dans laquelle ladite étape de modification de ladite interaction moléculaire prédéfinie comprend une étape pour le changement d'une température de ladite couche superficielle (4).

**13.** Méthode selon l'une quelconque des revendications 9 à 12, dans laquelle ladite étape de modification de ladite interaction moléculaire prédéfinie comprend une étape pour l'application d'un champ électrique et/ou magnétique orienté perpendiculairement ou à un certain angle par rapport à ladite surface dudit support (5).

**14.** Méthode selon l'une quelconque des revendications 9 à 13, dans laquelle ladite étape de modification de ladite interaction moléculaire prédéfinie comprend une étape pour l'excitation du polymère par la lumière.

**15.** Méthode selon l'une quelconque des revendications précédentes, dans laquelle ladite force externe est fournie en utilisant l'un des champs suivants : champ électrique, champ magnétique, champ optique et champ mécanique, ou toute combinaison de ces derniers.

**16.** Méthode selon l'une quelconque des revendications précédentes, dans laquelle un microscope à sonde de balayage SPM est utilisé pour l'application de ladite force externe.

**17.** Méthode selon l'une quelconque des revendications précédentes, dans laquelle la molécule polymère (1) comprend un polynucléotide comme un ADN ou ARN, un polypeptide comme une protéine, un anticorps ou système antigène-anticorps, un polysaccharide, ou un mélange souhaité de biomolécules.

**18.** Méthode selon l'une quelconque des revendications précédentes, dans laquelle ladite couche superficielle (4) comprend un polymère inorganique, un polymère organique, un métal, une substance organique de faible poids moléculaire, un oxyde métallique, un sulfure, un semi-conducteur, ou un élément optique, ou toute combinaison de ces derniers.

**19.** Méthode selon l'une quelconque des revendications précédentes, dans laquelle ledit substrat (5) est atomiquement plat.

**20.** Méthode selon l'une quelconque des revendications précédentes, dans laquelle ledit substrat (5) comprend du verre, du silicone oxydé en surface, de l'or, du sulfure de molybdène, du graphite pyrolytique hautement orienté (HOPG) ou du mica.

**21.** Méthode selon l'une quelconque des revendications précédentes, dans laquelle la méthode comprend une étape pour l'ancrage d'au moins une extrémité de la molécule polymère (1) sur ledit support (5).

**22.** Méthode selon l'une quelconque des revendications précédentes, dans laquelle la méthode comprend une étape pour l'ancrage d'au moins une extrémité de la molécule polymère (1) de manière à former une fibre, une microparticule ou une nanoparticule.

**23.** Utilisation d'une méthode selon l'une quelconque des revendications 1 à 22 pour la reconnaissance, la détection ou l'analyse d'un composant de milieu environnant et/ou d'une molécule polymère (1) à arranger.

**24.** Utilisation d'une méthode selon l'une quelconque des revendications 1 à 22 pour la reconnaissance, la détection ou l'analyse d'une molécule ou de groupes chimiques d'une couche superficielle (4).

**25.** Utilisation d'une méthode selon l'une quelconque des revendications 1 à 22 pour la construction d'un dispositif moléculaire.

Interaction with medium

Fig.1

Applied external force

21

Polymer
segment

**1st fixed conformation** | Polymer

20

Movement of first segment

Manipulation

Movement of 2nd and 3rd segments

Release of force

**2nd fixed conformation**

22

Fig. 2

Stretching:

Stretched DNA

Overstretching:

External force

Overstretched DNA

Fig. 3

# DNA on $C_{12}H_{25}NH_2$

Fig. 4

fig. 5

fig. 6

Weak complex formation, isolated
single polymer molecules on the surface

Strong complex formation, dense
package of aligned polymer
molecules on the support

d - period of crystalline 2dim matrix

Fig. 7

Fig. 8

Fig.9 A

Fig. 9B

40° C        50° C

Fig. 10

60°C

*Fig.* 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6376177 B
- US 5851769 A
- US 6303296 B
- WO 9706278 A
- US 5612181 A
- US 5314829 A
- US 5677126 A
- US 5079169 A

### Non-patent literature cited in the description

- **L. SHU et al.** *Angew. Chem.,* 2001, vol. 113, 4802
- **R.H. AUSTIN et al.** *Stretch genes, Physics Today,* 1997, vol. 2, 32-38
- **W.D. VOLKMUTH ; R.H. AUSTIN.** *Nature,* 1992, vol. 358, 600
- **SMITH et al.** *Science,* 1992, vol. 258, 1122
- **T. KANNO et al.** *Appl. Phys. Lett.,* 2000, vol. 77, 3848
- **NAKAO.** *Nano Letters,* 2002, vol. 2, 475
- **HU, J. et al.** *Artifical DNA Patterns by Mechanical Nanomanipulation,* January 2002, vol. 2 (1), 55-57
- **P.J. FLORY.** Principles of Polymer Chemistry. Cornell University Press, 1995
- **M. RIEF et al.** *Science,* 1997, vol. 275, 1295